(19) Europäisches Patentamt / European Patent Office / Office européen des brevets

(11) **EP 2 144 620 B1**

(12) **FASCICULE DE BREVET EUROPEEN**

(45) Date de publication et mention
de la délivrance du brevet:
**21.10.2015 Bulletin 2015/43**

(21) Numéro de dépôt: **08805723.7**

(22) Date de dépôt: **28.04.2008**

(51) Int Cl.:
***A61K 36/185*** *(2006.01)*

(86) Numéro de dépôt international:
**PCT/FR2008/050769**

(87) Numéro de publication internationale:
**WO 2008/145931 (04.12.2008 Gazette 2008/49)**

(54) **NOUVEAU PROCEDE DE PREPARATION D'EXTRAITS PURIFIES D'HARPAGOPHYTUM PROCUMBENS**

NEUARTIGES VERFAHREN ZUR HERSTELLUNG GEREINIGTER EXTRAKTE AUS HARPAGOPHYTUM PROCUMBENS

NOVEL METHOD FOR PREPARING PURIFIED EXTRACTS OF HARPAGOPHYTUM PROCUMBENS

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **07.05.2007 FR 0754906**

(43) Date de publication de la demande:
**20.01.2010 Bulletin 2010/03**

(73) Titulaire: **Naturex**
**84140 Avignon (FR)**

(72) Inventeurs:
• **ROLLAND, Yohan**
**F-71000 Macon (FR)**
• **DUVAL, Charles**
**F-71850 Charnay Les Macon (FR)**

(74) Mandataire: **Marek, Pierre**
**Cabinet Marek**
**28, rue de la Loge**
**13235 Marseille Cédex 02 (FR)**

(56) Documents cités:
**WO-A-97/44051       DE-A1- 19 603 788
DE-A1- 19 651 290    US-A1- 2002 183 264
US-B1- 6 280 737**

• **DATABASE WPI Week 199309 Thomson Scientific, London, GB; AN 1993-073947 XP002505231 & KR 920 005 686 B (DONGKUK PHARM CO) 13 juillet 1992 (1992-07-13)**
• **SCHMIDT A H: "Fast HPLC for quality control of Harpagophytum procumbens by using a monolithic silica column: method transfer from conventional particle-based silica column" JOURNAL OF CHROMATOGRAPHY A, ELSEVIER, AMSTERDAM, NL, vol. 1073, no. 1-2, 6 mai 2005 (2005-05-06), pages 377-381, XP004850593 ISSN: 0021-9673**
• **GÜNTHER M ET AL: "High anti-inflammatory activity of harpagoside-enriched extracts obtained from solvent-modified super- and subcritical carbon dioxide extractions of the roots of Harpagophytum procumbens" PHYTOCHEMICAL ANALYSIS, XX, XX, vol. 17, no. 1, janvier 2006 (2006-01), pages 1-7, XP009092504**
• **FAIVRE C ET AL: "Grapple plant or devil's claw. Harpagophytum procumbens (Pedaliaceae)" PHYTOTHERAPIE, SPRINGER-VERLAG, PARIS, FR, vol. 5, no. 3, 2007, pages 150-153, XP009092503 ISSN: 1624-8597**

## Description

La présente invention a pour objet un procédé de préparation d'extraits purifiés *d'Harpagophytum procumbens,* lesdits extraits étant sous forme liquide ou sèche.

La présente invention a également pour objet des extraits purifiés tels qu'obtenus par ledit procédé.

L'Harpagophytum (*Harpagophytum procumbens*) est une plante originaire d'Afrique du Sud, traditionnellement reconnue pour son efficacité dans le traitement, entre autres, des douleurs de l'arthrose.

Ses racines secondaires tubérisées renferment en particulier des composés de type iridoïdes, glycosylés ou non, considérés comme les principes actifs, nommés harpagoside, harpagide, procumbide et procumboside.

La phytothérapie moderne utilise dans des galéniques en forme sèche de type gélules, de la poudre de plante ou des extraits de la plante. L'utilisation de poudre de plante impose un très grand nombre de prises par jour ce qui est très contraignant au quotidien.

Les industries des compléments alimentaires et de la phytothérapie disposent maintenant d'un grand nombre d'extraits de plante pour ces applications.

Les extraits d'harpagophytum sont obtenus usuellement en utilisant des solvants hydro-alcooliques, le titre alcoolique pouvant varier de 0 (extrait à l'eau) jusqu'à 90 % (Chrubasik S. ; Devil's claw extract as an example of the effectiveness of herbal analgesics; Orthopade. 2004 Jul;33(7):804-8).

La Pharmacopée Européenne décrit également dans la monographie de la racine d'harpagophytum l'utilisation de méthanol pour l'extraction de l'harpagoside.

La plante est séparée du solvant d'extraction par tous les procédés habituels, par exemple de type filtration ou centrifugation. Le filtrat est désolvantisé dans le cas de l'utilisation de solvant alcoolique, éventuellement concentré pour réduire le volume, puis séché.

Ce type de procédé permet d'obtenir des extraits d'harpagophytum titrés à 2,5 - 3 % d'harpagoside, mesuré par HPLC. Par ailleurs, la variation des paramètres usuels de l'extraction végétale comme la nature du solvant, la température et la durée d'extraction, n'ont pas d'impact sur le titre final de la poudre obtenue. On obtient toujours un titre autour de 2,5 - 3 %.

Les produits qui titrent 2,5 à 3% d'harpagoside par HPLC offrent une alternative satisfaisante à l'usage de poudre de plante pour la réalisation de gélule, mais ne permettent pas de réduire significativement la posologie pour les traitements pour lesquels l'harpagophytum est efficace.

Seuls des produits plus titrés en harpagoside pourraient limiter de façon significative la prise quotidienne de gélules.

Il manque donc un procédé qui permet d'obtenir industriellement des extraits d'harpagophytum en poudre avec des hauts titres en harpagoside.

Parmi les moyens décrits pour obtenir un tel résultat, et bien que, compte tenu de la polarité de la molécule, l'homme de l'art ne soit pas amené immédiatement à cette solution, on peut citer l'utilisation de fluide super ou sub-critique (Gunther M, Laufer S, Schmidt PC. High anti-inflammatory activity of harpagoside-enriched extracts obtained from solvent-modified super- and subcritical carbon dioxide extractions of the roots of Harpagophytum procumbens; Phytochem Anal. 2006 Jan-Feb;17(1):1-7).

Des extractions au dioxyde de carbone / solvant modifié de racines de *Harpagophytum procumbens* ont été étudiées en ce qui concerne l'efficacité d'extraction et le contenu en harpagoside, et comparées à un extrait conventionnel.

Les effets de la pression, de la température, du type et de la concentration du modificateur ont été examinés. Deux étapes d'extraction étaient nécessaires pour obtenir des extraits anti-inflammatoires fortement enrichis en harpagoside. La première étape d'extraction a été effectuée dans l'état supercritique en utilisant le dioxyde de carbone modifié avec du n-propanol pour enlever les substances lipophiles peu désirées. L'extraction principale a été exécutée dans l'état supercritique ou sub-critique avec du dioxyde de carbone modifié avec de l'éthanol. L'extraction liquide supercritique a eu comme conséquence des extraits contenant l'harpagoside jusqu'à plus de 30%. Les extraits sub-critiques ont montré une teneur en harpagoside de 20%, mais le rendement d'extraction était presque trois fois plus grand que dans des conditions supercritiques.

Le taux de recouvrement en harpagoside résultant de la somme de l'extrait et du résidu brut était de 99% plus grand que dans toutes les expériences précédentes. L'extrait conventionnel et deux extraits au dioxyde de carbone ont été examinés pour l'inhibition *in vitro* de la lipoxygénase 5 ou de la biosynthèse cyclooxygénase-2. Les deux extraits au dioxyde de carbone ont montré l'inhibition totale sur la biosynthèse de la lipoxygénase 5 à une concentration de 51,8 mg/l ; en revanche, l'extrait conventionnel ne montre pas d'inhibition de biosynthèse de la lipoxygénase 5.

On montre bien ici l'intérêt des extraits purifiés de racine d'harpagophytum. Cependant il faut replacer ce type d'extraits au $CO_2$ super ou sub-critique dans un contexte industriel. Les extraits obtenus par $CO_2$ sont dans un ordre de prix très élevé, beaucoup plus élevé que les extraits par solvant, et ce type de procédés nécessite des installations très importantes, compte tenu des très fortes pressions employées.

Les produits obtenus par les extractions au $CO_2$ ne sont généralement pas en poudre, mais plutôt sous forme de pâte ou de cire, ce qui n'est pas compatible avec la fabrication de gélules par exemple, ou alors éventuellement adsorbés sur un support, ce qui diminue le titre du produit. Par ailleurs, les produits ainsi obtenus sont des extraits liposolubles et donc non solubles dans les phases aqueuses. Enfin, on peut signaler que ce type de procédé utilisant le $CO_2$ entraîne les composés de type pesticides et mycotoxines, donc les concentrent dans le produit fini. Ainsi, les extractions au $CO_2$ ne permettent pas d'obtenir des extraits d'harpagophytum présentant un titre élevé d'harpagoside compatibles avec toutes les formulations galéniques liquides ou sèches.

Ainsi, la présente invention a pour but de fournir un procédé de préparation industriellement applicable d'extraits purifiés d'harpagophytum présentant un titre élevé en harpagoside, lesdits extraits ainsi obtenus étant compatibles avec toutes les galéniques liquides (sirops ou capsules) ou sèches (comprimés ou gélules).

La présente invention a également pour but de fournir des extraits d'harpagophytum fortement titrés en harpagoside et solubles dans les phases aqueuses.

La présente invention concerne un procédé de préparation d'un extrait concentré d'*Harpagophytum procumbens,* sous forme liquide ou sèche, avec un titre en harpagoside supérieur ou égal à 5%, de préférence supérieur ou égal à 35%, comprenant une étape de purification d'un extrait brut d'*Harpagophytum procumbens* sous forme liquide en phase aqueuse, par une technique d'extraction liquide-liquide avec un solvant organique choisi parmi les esters, notamment les esters aliphatiques, et plus particulièrement parmi les acétates d'alkyle, ledit groupe alkyle étant une chaîne alkylée, ramifiée ou linéaire, comprenant de 1 à 10, notamment de 1 à 6, et préférentiellement de 1 à 4 atomes de carbone.

Ainsi, la présente invention est basée sur le fait que les inventeurs ont constaté que les iridoïdes glucosides de l'harpagophytum peuvent être de façon surprenante purifiés à l'aide de solvants de type esters.

En effet, à ce jour, on connaît l'utilisation de solvants tels que les alcools et les cétones.

Même si la polarité selon Snyder classe l'acétate d'éthyle (4,4) au même rang que l'éthanol (4,3), mais plus bas que le méthanol (5,1) et l'acétone (5,1), et légèrement supérieur au butanol (3,9), la constante diélectrique de l'acétate d'éthyle ($\varepsilon$ = 6) est extrêmement faible et ne permet pas du tout de supposer que l'acétate d'éthyle puisse créer des liaisons d'affinité avec des molécules polaires, donc de les solubiliser, et a fortiori de purifier des molécules du type iridoïdes glucosides.

De façon surprenante, les esters tels que l'acétate d'éthyle sont très sélectifs par rapport à ce type de composés, et permettent des purifications très efficaces jusqu'à des titres supérieurs à 35% sur sec.

Parmi tous les écueils rencontrés lors de la fabrication d'un extrait d'harpagophytum, il y a la difficulté liée à la dégradation des principes actifs. Ainsi, l'homme du métier utilise les solvants tels que l'eau ou des alcools pour réaliser les extraits bruts, et des solvants tels que des alcools et des cétones aliphatiques pour les purifier.

Si ces extraits sont réalisés dans les conditions habituelles (extraction, élimination du solvant organique sous vide si besoin, concentration, pour obtenir un extrait brut, qui peut être séché ou purifié à l'aide d'un autre solvant organique, avec élimination du solvant organique sous vide, concentration et séchage), on obtient toujours des titres inférieurs à 3% en harpagoside. Les produits intermédiaires sont bien plus concentrés, mais les différentes étapes entraînent une dégradation progressive des principes actifs.

Parmi les améliorations proposées, on peut citer le séchage en phase solvant organique (alcool ou butanol) (tel que décrit par exemple dans le brevet US 6 280 737) qui nécessite des équipements très coûteux et peu performants. Dans tous les cas, les titres en harpagoside des extraits obtenus ainsi sont au maximum de 20%.

L'utilisation d'un solvant organique non miscible à l'eau de type butanol pour la purification des extraits d'harpagophytum pose également le problème de la température imposée au produit pour éliminer ledit solvant organique. En effet, le butanol seul bout à 117°C à température ambiante et le mélange eau-butanol rencontré pendant les étapes de purification bout à 92,6°C. Même en condition d'évaporation - séchage sous vide, le produit est alors soumis à une forte température, ce qui entraîne une dégradation des actifs.

L'utilisation d'un solvant organique non miscible à l'eau de type ester permet de réaliser les opérations d'évaporation - séchage à une température beaucoup plus faible, et donc de limiter les dégradations des principes actifs. Ainsi, l'utilisation d'esters dont le point d'ébullition est inférieur à celui des alcools utilisés dans l'état de la technique (notamment le butanol) permet de ne pas exposer le produit (principe actif - harpagoside) à des hautes températures, ce qui permet de réduire la durée du procédé exposant le produit à des températures élevées. Les dégradations thermiques des principes actifs n'ont alors pas lieu et on peut ainsi conserver des titres élevés sur les extraits purifiés obtenus (jusqu'à au moins 35%).

Par ailleurs, du fait de la possibilité de concentrer le produit à basse température, on peut réaliser un concentré en phase aqueuse débarrassé de la fraction solvant organique, et gagner en productivité sur l'étape de séchage qui peut se faire sur des équipements standards donc moins onéreux, donc mieux dimensionnés. La lyophilisation peut être un moyen de séchage utilisé par exemple.

Le titre en harpagoside mentionné ci-dessus est un titre poids/poids (p/p) exprimé par rapport au poids total de matières sèches.

Il est mesuré par la méthode de la pharmacopée Européenne 5.07 comme suit : on opère par chromatographie liquide en utilisant le cinnamate de méthyle comme étalon interne.

*Préparation de la solution d'étalon interne :* on dissout 0,130 g de cinnamate de méthyle dans 50 ml de méthanol et on complète à 100,0 ml avec le même solvant.

*Préparation de la solution à analyser :* à 0,500 g de racine d'harpagophyton (*Harpagophytum procumbens*) pulvérisée, on ajoute 50 ml de méthanol. On agite pendant 1 h et on filtre. Le résidu est ensuite transféré dans un ballon de 100 ml, et on ajoute 50 ml de méthanol et on chauffe à reflux pendant 1 h. L'ensemble est alors refroidi et filtré. Le ballon et le filtre sont alors lavés avec 2 fois 5 ml de méthanol. Ensuite, les extraits sont réunis et évaporés à siccité sous pression réduite et à une température ne dépassant pas 40°C. Puis le résidu est traité avec 3 fois 5 ml de méthanol et les extraits sont filtrés dans une fiole jaugée de 25 ml. En lavant le filtre, on complète à 25,0 ml avec du méthanol. A 10,0 ml de cette solution, on ajoute 1,0 ml de solution d'étalon interne et on complète à 25,0 ml avec du méthanol.

*Préparation de la solution témoin :* On prélève 0,5 ml de solution témoin (1 mg d'harpagoside dans 1 ml de méthanol) et on complète à 2,0 ml avec du méthanol.

La chromatographie peut être par exemple réalisée en utilisant :

- une colonne d'acier inoxydable, d'une longueur de 0,10 m et d'un diamètre intérieur de 4 mm, remplie de gel de silice octadécylsilylé pour chromatographie (5 $\mu$m),
- comme phase mobile, à un débit de 1,5 ml/min, un mélange à volumes égaux d'eau et de méthanol,
- comme détecteur, un spectrophotomètre réglé à 278 nm,
- et un injecteur à boucle de 10 $\mu$l.

*Analyse par chromatographie :* la solution à examiner est injectée et on ajuste la sensibilité du système de façon que la hauteur du pic correspondant au cinnamate de méthyle représente 50% de l'échelle totale de l'enregistreur.

Le temps de rétention de l'harpagoside est déterminé en utilisant 10 $\mu$l de solution témoin examinée dans les mêmes conditions que la solution à examiner. Ensuite, la teneur (%) en harpagoside est calculée à l'aide de l'expression

$$\frac{m_2 \times F_1 \times 7,622}{F_2 \times m_1}$$

où

$m_1$ = masse de la prise d'essai (g)
$m_2$ = masse du cinnamate de méthyle (g) dans la solution d'étalon interne
$F_1$ = surface du pic correspondant à l'harpagoside dans le chromatogramme obtenu avec la solution à analyser
$F_2$ = surface du pic correspondant au cinnamate de méthyle dans le chromatogramme obtenu avec la solution à analyser

Selon un mode de réalisation particulier, la présente invention concerne un procédé de préparation d'un extrait concentré d'*Harpagophytum procumbens,* sous forme liquide ou sèche, avec un titre en harpagoside supérieur ou égal à 5%, comprenant les étapes suivantes :

- une étape de purification de type extraction liquide-liquide entre un extrait brut d'*Harpagophytum procumbens* sous forme liquide en phase aqueuse et un solvant organique choisi parmi les esters, pour obtenir une phase aqueuse et une phase organique concentrée en harpagoside,
- une étape d'élimination du solvant à partir de la phase organique précédemment obtenue, pour obtenir ledit extrait concentré sous forme liquide, et
- une éventuelle étape de transformation dudit extrait concentré sous forme liquide par des moyens physiques ou physico-chimiques, pour obtenir un extrait concentré sous forme sèche.

La phase organique concentrée en harpagoside présente de préférence un titre en harpagoside supérieur ou égal à 5%.

Selon un mode de réalisation avantageux de la présente invention, le solvant organique utilisé dans le cadre de l'étape de purification par une technique de type extraction liquide-liquide est choisi parmi les esters, notamment les esters aliphatiques, et plus particulièrement parmi les acétates d'alkyle en $C_1$-$C_6$, de préférence $C_1$-$C_4$.

De préférence, le solvant organique utilisé pour l'étape d'extraction liquide-liquide est l'acétate de méthyle, l'acétate d'éthyle et l'acétate de butyle.

L'acétate d'éthyle est particulièrement préféré car il est le plus courant au niveau industriel, a une température d'ébullition très basse (77,06°C) et est un solvant alimentaire. Ainsi, l'acétate d'éthyle présente l'avantage d'être un solvant non miscible à l'eau autorisé en alimentaire qui permette de purifier les extraits d'harpagophytum et d'atteindre des titres supérieurs ou égaux à 35%.

L'utilisation de l'acétate d'éthyle permet de réaliser les opérations d'évaporation - séchage à une température beaucoup plus faible que lorsqu'on utilise par exemple le butanol. Cette diminution de la température permet alors de limiter les dégradations des principes actifs. Ainsi, l'acétate d'éthyle (point d'ébullition 77°C à la pression atmosphérique) et l'eau forment un azéotrope qui bout à 70,4°C à pression atmosphérique, et à moins de 40°C sous vide de 0,1 bar.

De plus l'azéotrope eau-butanol contient 42,4% d'eau, alors que l'azéotrope eau-acétate d'éthyle ne contient que 8,5% d'eau, ce qui permet son recyclage industriel pour de nouvelles purifications sans traitement supplémentaire.

La sélectivité de l'acétate d'éthyle par rapport aux molécules recherchées, iridoïdes glucosides de l'harpagophytum, ainsi que les propriétés dudit solvant à l'évaporation permettent d'obtenir des extraits à très hauts titres (> 35 %), avec une réelle performance économique industrielle, pour la réalisation de produit destiné aux compléments alimentaires.

Dans le cadre de l'étape de purification par extraction liquide-liquide, les équipements utilisés sont de tout type permettant le mélange de deux liquides, plus particulièrement réacteur agité ou colonne à garnissage et en particulier de type centrifuge, équipement qui permet de récolter rapidement les deux phases du procédé : la phase aqueuse concentrée, épuisée en harpagoside, et la phase organique chargée en harpagoside. Ce procédé permet de récupérer plus de 90% des principes actifs présents dans la phase aqueuse de départ.

Ainsi, à partir d'un extrait brut sous forme liquide en phase aqueuse, obtenu soit par extrait brut de la plante à l'eau, soit par extrait brut à l'alcool et désolvantisé, soit par remise en solution de l'extrait brut en poudre dans de l'eau, on peut appliquer une méthode de purification par extraction liquide-liquide.

La présente invention concerne également un procédé tel que défini ci-dessus, caractérisé en ce que l'extrait brut d'*Harpagophytum procumbens* est obtenu par un procédé comprenant une étape de mise en contact d'un solvant de type hydro-alcoolique constitué jusqu'à 90% d'alcool avec des racines secondaires séchées d'*Harpagophytum procumbens,* et notamment par extraction, macération, décoction ou percolation, une étape d'élimination du solvant par séparation solide/liquide, notamment par filtration, par désolvantisation ou par centrifugation pour récupérer ledit extrait brut et une éventuelle étape de concentration dudit extrait brut afin d'obtenir ledit extrait brut sous forme d'un liquide épais contenant de 2 à 50% en matières sèches.

De préférence, l'alcool mentionné ci-dessus constituant le solvant de type hydro-alcoolique est du méthanol ou de l'éthanol.

La présente invention concerne également un procédé tel que défini ci-dessus, dans lequel la transformation de l'extrait concentré sous forme liquide par des moyens physiques ou physico-chimiques est effectuée par élimination de l'eau dans un flux d'air chaud, par séchage, par nébulisation, par évaporation, par sublimation, par déshydratation, par adsorption sur support.

De préférence, la présente invention concerne un procédé de préparation d'un extrait concentré d'*Harpagophytum procumbens,* sous forme liquide, avec un titre en harpagoside supérieur ou égal à 5%, de préférence compris de 35 à 50%, ledit procédé comprenant :

- une étape de purification de type extraction liquide-liquide entre un extrait brut d'*Harpagophytum procumbens* sous forme liquide en phase aqueuse et un solvant organique choisi parmi les esters, pour obtenir une phase aqueuse et une phase organique concentrée en harpagoside, et
- une étape d'élimination du solvant à partir de la phase organique précédemment obtenue, pour obtenir ledit extrait concentré sous forme liquide.

De préférence, la présente invention concerne un procédé de préparation d'un extrait concentré d'*Harpagophytum procumbens,* sous forme sèche, avec un titre en harpagoside supérieur ou égal à 5%, de préférence compris de 35 à 50%, ledit procédé comprenant :

- une étape de purification de type extraction liquide-liquide entre un extrait brut d'*Harpagophytum procumbens* sous forme liquide en phase aqueuse et un solvant organique choisi parmi les esters, pour obtenir une phase aqueuse et une phase organique concentrée en harpagoside,
- une étape d'élimination du solvant à partir de la phase organique précédemment obtenue, pour obtenir ledit extrait concentré sous forme liquide, et
- une étape de transformation dudit extrait concentré sous forme liquide par des moyens physiques ou physico-chimiques, pour obtenir un extrait concentré sous forme sèche.

La présente invention concerne également les extraits tels qu'obtenus par la mise en oeuvre du procédé de l'invention tel que défini ci-dessus.

La présente invention concerne un extrait concentré d'*Harpagophytum procumbens,* sous forme liquide, soluble en phase aqueuse, avec un titre en harpagoside supérieur ou égal à 35%, de préférence compris de 35% à 50%, notamment de 35% à 45%, et préférentiellement de 35% à 40%.

La présente invention concerne également un extrait concentré d'*Harpagophytum procumbens,* sous forme sèche, avec un titre en harpagoside supérieur ou égal à 35%, de préférence compris de 35% à 50%, notamment 35% à environ 45%, et préférentiellement de 35% à 40%.

La présente invention concerne également une composition pharmaceutique comprenant à titre de substance active un extrait concentré d'*Harpagophytum procumbens,* sous forme liquide ou sèche, en association avec un véhicule pharmaceutiquement acceptable.

La présente invention concerne également un complément alimentaire comprenant un extrait concentré d'*Harpagophytum procumbens,* sous forme liquide ou sèche, en association avec un véhicule acceptable.

EXEMPLES

EXEMPLE 1

*1) Réalisation de l'extrait brut :*

L'extrait brut est préparé à partir d'une tonne de racines secondaires d'harpagophytum. Ces racines secondaires sont mises à macérer dans l'eau, sous agitation, pendant 2 heures. Ensuite, on effectue une étape de séparation entre la plante et le solvant par filtrations successives (600 $\mu$m, 100 $\mu$m, puis 25 $\mu$m). L'étape suivante est une étape de concentration sous vide ce qui permet d'éliminer encore plus de solvant et permet d'éviter de manipuler par la suite une trop grande quantité de solvant.

On obtient alors un extrait brut sous forme concentrée.

*2) Réalisation de la purifications:*

Le concentré est directement extrait en phase liquide avec 1 000 L d'acétate d'éthyle en utilisant un extracteur centrifuge en continu.

La phase organique est récupérée et désolvantisée sous vide.

On obtient un concentré en phase aqueuse qui peut être réduit en poudre par exemple en réacteur agité sous vide.

Le produit obtenu titre 35 % en harpagoside mesuré par HPLC. Il faut noter que la phase aqueuse initiale est épuisée en harpagoside et contient moins de 0,3% d'harpagoside.

EXEMPLE 2

*1) Réalisation de l'extrait brut :*

L'extrait brut est préparé à partir d'un kg de racines secondaires d'harpagophytum. Ces racines secondaires sont mises à macérer dans l'éthanol (70% vol.) à 65°C, sous agitation, pendant 2 heures. Ensuite, on effectue une étape de séparation entre la plante et le solvant par filtrations successives (600 $\mu$m, 100 $\mu$m, puis 25 $\mu$m). L'étape suivante est une étape de concentration sous vide ce qui permet d'éliminer encore plus de solvant et permet d'éviter de manipuler par la suite une trop grande quantité de solvant.

On obtient alors un extrait brut sous forme concentrée (200 mL).

*2) Réalisation de la purification :*

Le concentré est directement extrait en phase liquide avec 1 000 mL d'acétate d'éthyle en utilisant un extracteur centrifuge en continu (à 0°C).

La phase organique est récupérée et désolvantisée sous vide.

On obtient un concentré en phase aqueuse qui peut être réduit en poudre par exemple par lyophilisation. Ainsi, dans le cadre de cet exemple, on obtient une poudre (20 g).

Le produit obtenu titre 55 % en harpagoside mesuré par HPLC.

**Revendications**

1. Procédé de fabrication d'un extrait concentré *d'Harpagophytum procumbens,* sous forme liquide ou sèche avec un titre en harpagoside supérieur ou égal à 5 %, comprenant une étape de purification d'un extrait brut *d'Harpagophytum procumbens* sous forme liquide en phase aqueuse, par une technique d'extraction liquide-liquide avec un solvant organique choisi parmi les esters, de préférence les esters aliphatiques, et plus particulièrement les acétates d'alkyle.

2. Procédé selon la revendication 1, caractérisé en ce qui le solvant organique est de l'acétate d'éthyle.

3. Procédé selon la revendication 1 ou 2, comprenant les étapes suivantes :

   - une étape de purification de type extraction liquide-liquide entre extrait brut *d'Harpagophytum procumbens* sous forme liquide en phase aqueuse et un solvant organique choisi parmi les esters, pour obtenir une phase aqueuse et une phase organique concentrée en harpagoside,
   - une étape d'élimination du solvant à partir de la phase organique précédemment obtenue, pour obtenir ledit extrait concentré sous forme liquide, et
   - une éventuelle étape de transformation dudit extrait concentré sous forme liquide par des moyens physiques ou physico-chimiques, pour obtenir un extrait concentré sous forme sèche.

4. Procédé selon l'une quelconque des revendications 1 à 3, **caractérisé en ce que** l'extrait brut *d'Harpagophytum procumbens* utilisé pour la mise en oeuvre de l'étape de purification est obtenu par un procédé comprenant une étape de mise en contact d'un solvant de type hydro-alcoolique constitué jusqu'à 90 % d'alcool avec des racines secondaires séchées *d'Harpagophytum procumbens,* et notamment par extraction, macération, décoction ou percolation, une étape d'élimination du solvant par séparation solide/liquide, notamment par filtration, par désolvantisation ou par centrifugation pour récupérer ledit extrait brut et une éventuelle étape de concentration dudit extrait brut afin d'obtenir ledit extrait brut sous forme d'un liquide contenant de 2 à 50 % en matières sèches.

5. Procédé selon la revendication 4, **caractérisé en ce que** l'alcool constituant le solvant de type hydro-alcoolique est du méthanol ou de l'éthanol.

6. Procédé selon l'une quelconque des revendications 1 à 5, dans lequel la transformation de l'extrait concentré sous forme liquide par des moyens physiques ou physico-chimiques est effectuée par élimination de l'eau dans un flux d'air chaud, par séchage, par nébulisation, par évaporation, par sublimation, par déshydratation ou par adsorption su support.

7. Extrait concentré *d'Harpagophytum procumbens,* sous forme liquide, avec un titre en harpagoside supérieur ou égal à 35 %, de préférence compris de 35 % à 50 %, notamment de 35 % à 45 %, et préférentiellement de 35 % à 40 %.

8. Extrait concentré *d'Harpagophytum procumbens,* sous forme sèche, avec un titre en harpagoside supérieur ou égal à 35 %, de préférence compris de 35 % à 50 % , notamment de 35 % à 45 %, et préférentiellement de 35 % à 40 %.

9. Composition pharmaceutique comprenant à titre de substance active un extrait concentré *d'Harpagophytum procumbens* selon la revendication 7 ou 8, en association avec un véhicule pharmaceutiquement acceptable.

10. Complément alimentaire comprenant un extrait concentré *d'Harpagophytum procumbens* selon la revendication 7 ou 8, en association avec un véhicule acceptable.

**Patentansprüche**

1. Verfahren zur Herstellung eines konzentrierten Extrakts aus Harpagophytum procumbens, in flüssiger oder trockener Form, mit einem Harpagosid Titer größer als oder gleich 5%, umfassend einen Schritt der Reinigung eines rohen Extrakts aus Harpagophytum procumbens in flüssiger Form aus der flüssigen Phase durch eine Technik der Flüssig-Flüssig-Extraktion mit einem organischen Lösungsmittel ausgewählt unter den Estern, vorzugsweise den aliphatischen Estern und weiter bevorzugt aus Alkylacetaten.

2. Verfahren nach Anspruch 1, **dadurch gekennzeichnet, dass** das organische Lösungsmittel ein Alkylacetat ist.

3. Verfahren nach Anspruch 1 oder 2, umfassend die folgenden Schritte:

    - einen Reinigungsschritt des Typs der Flüssig-Flüssig-Extraktion zwischen einem rohen Extrakts aus Harpagophytum procumbens in flüssiger Form aus der flüssigen Phase und einem organischen Lösungsmittel ausgewählt unter den Estern um eine flüssige Phase und eine konzentrierte organische Phase Harpagosid,
    - einen Schritt der Beseitigung des Lösungsmittels ab der zuvor gewonnenen organischen Phase, um das genannte Extrakt in konzentrierter flüssiger Form zu erhalten und
    - einen eventuellen Schritt der Transformation des genannten konzentrierten Extrakts in der flüssigen Phase durch physikalische oder physikalischchemische Mittel, um einen konzentrierten Extrakt in trockener Form zu erhalten.

4. Verfahren nach einem der Ansprüche 1 bis 3, **dadurch gekennzeichnet, dass** das rohe Extrakt aus Harpagophytum procumbens, das zur Durchführung des Reinigungsschrittes verwendet wurde, durch ein Verfahren, umfassen einen Schritt des In-Kontaktbringens mit einem Wasser-Alkohol-Lösemitteltyp, der bis zu 90% Alkohol umfasst mit trockenen sekundären Wurzeln aus Harpagophytum procumbens und insbesondere durch Extraktion, Mazeration, Auskochen oder Perkolationsverfahren erhalten wurde, und einen Schritt der Beseitigung des Lösemittels durch eine fest/flüssig Separation, insbesondere durch Filtern, durch Lösemittelentfernung oder durch Zentrifugieren um das genannte rohe Extrakt zurückzugewinnen und einen eventuellen Schritt der Konzentrierung des genannten rohen Extrakts, um das genannte rohe Extrakt in einer flüssigen Form umfassend 2 bis 20% Trockenmasse zu gewinnen.

5. Verfahren nach Anspruch 4, **dadurch gekennzeichnet, dass** der Alkohol, der das Lösungsmittel vom Typ der Flüssig-Alkohole umfasst, Methanol oder Ethanol ist.

6. Verfahren nach einem der Ansprüche 1 bis 5, wobei die Transformation des konzentrierten Extrakts in flüssige Form durch physikalische oder physikalisch-chemische Mittel durch Beseitigung des Wassers in einem warmen Luftstrom, durch Trocknung, durch Vernebelung, durch Verdunstung, durch Sublimation, durch Dehydration oder durch bekannte Adsorptionsverfahren bewirkt wird.

7. Konzentriertes Extrakt aus Harpagophytum procumbens, in flüssiger Form, mit einem Harpagosid Titer größer als oder gleich 5%, vorzugsweise von 35% bis 50%, insbesondere von 35% bis 45% und bevorzugt von 35% bis 40%.

8. Konzentriertes Extrakt aus Harpagophytum procumbens, in trockener Form, mit einem Harpagosid Titer größer als oder gleich 5%, vorzugsweise von 35% bis 50%, insbesondere von 35% bis 45% und bevorzugt von 35% bis 40%.

9. Pharmazeutische Zusammensetzung enthaltend einen konzentrierten Wirkstoff aus Harpagophytum procumbens gemäß dem Anspruch 7 oder 8 in Verbindung mit einem pharmazeutisch aufnehmbaren Träger.

10. Nahrungsergänzungsmittel enthaltend einen konzentrierten Wirkstoff aus Harpagophytum procumbens gemäß dem Anspruch 7 oder 8 in Verbindung mit einem aufnehmbaren Träger.

**Claims**

1. A process for the production of a concentrated extract of *Harpagophytum procumbens* in liquid or dry form with a harpagoside titre of greater than or equal to 5%, comprising a step for purification of a crude extract of *Harpagophytum procumbens* in liquid form in aqueous phase by a procedure of liquid-liquid extraction with an organic solvent selected from esters, preferably aliphatic esters, more particularly alkyl acetates.

2. A process according to claim 1 **characterised in that** the organic solvent is ethyl acetate.

3. A process according to claim 1 or claim 2 comprising the following steps:

   - a purification step of liquid-liquid extraction type between a crude extract of *Harpagophytum procumbens* in liquid form in aqueous phase and an organic solvent selected from esters to obtain an aqueous phase and a harpagoside-concentrated organic phase,
   - a step for elimination of the solvent from the previously obtained organic phase to obtain said concentrated extracted liquid form, and
   - an optional step for transformation of said concentrated extract in liquid form by physical or physico-chemical means to obtain a concentrated extract in dry form.

4. A process according to any one of claims 1 to 3 **characterised in that** the crude extract of *Harpagophytum procumbens* used for carrying out the purification step is obtained by a process comprising a step in which a solvent of hydro-alcohol type constituted by up to 90% of alcohol with dried secondary roots of *Harpagophytum procumbens* and in particular by extraction, maceration, decoction or percolation, a step for removal of the solvent by solid/liquid separation, in particular by filtration, by desolvantisation or by centrifuging to recover said crude extract and an optional step of concentrating said crude extract to obtain said crude extract in the form of a liquid containing 2 to 50% of dry matter.

5. A process according to claim 4 **characterised in that** the alcohol constituting the solvent of hydro-alcohol type is methanol or ethanol.

6. A process according to any one of claims 1 to 5 wherein transformation of the concentrated extract in liquid form by physical or physico-chemical means is effected by elimination of the water in a hot air flow, by drying, by nebulisation, by evaporation, by sublimation, by dehydration or by adsorption on a support.

7. A concentrated extract of *Harpagophytum procumbens* in liquid form with a harpagoside titre of greater than or equal to 35%, preferably from 35% to 50%, in particular from 35% to 45% and preferably from 35% to 40%.

8. A concentrated extract of *Harpagophytum procumbens* in dry form with a harpagoside titre of greater than or equal to 35%, preferably from 35% to 50%, in particular from 35% to 45% and preferably from 35% to 40%.

9. A pharmaceutical composition comprising as the active ingredient a concentrated extract of *Harpagophytum procumbens* according to claim 7 or claim 8 in association with a pharmaceutically acceptable carrier.

10. A food supplement comprising a concentrated extract of *Harpagophytum procumbens* according to claim 7 or claim 8 in association with an acceptable carrier.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6280737 B

**Littérature non-brevet citée dans la description**

- **CHRUBASIK S.** Devil's claw extract as an example of the effectiveness of herbal analgesics. *Orthopade,* Juillet 2004, vol. 33 (7), 804-8

- **GUNTHER M ; LAUFER S ; SCHMIDT PC.** High anti-inflammatory activity of harpagoside-enriched extracts obtained from solvent-modified super- and subcritical carbon dioxide extractions of the roots of Harpagophytum procumbens. *Phytochem Anal.,* Janvier 2006, vol. 17 (1), 1-7